# EUROPEAN PATENT APPLICATION

(11) **EP 1 598 709 A2**
(43) Date of publication of application: **23.11.2005**
(21) Application number: 05252962.5
(22) Date of filing: 13.05.2005
(51) Int. Cl.: G03G 15/32

(54) **Electrostatic latent image forming medium using TFT array and image forming apparatus including the electrostatic latent image forming medium**

(30) Priority: 19.05.2004 KR 2004035548
(71) Applicant: SAMSUNG ELECTRONICS CO., LTD., Suwon-si, Gyeonggi-do 442-742 (KR)
(72) Inventor: Kim, Seong-jin, c/o 120-904 Park Town Samick Apt., Seongnam-si, Gyeonggi-do (KR); Shin, Seung-joo, c/o 1116-1506 Jugong Apt., Seoul (KR); Kwon, Kye-si, c/o 503-410 Banpo Mido 2-cha Apt., Seoul (KR); Kwon, Jang-yeon, c/o D-3403 Michelan Chereville, Seongnam-si, Gyeonggi-do (KR); Moon, Il-kwon, c/o 113-2004 Neulpureun Byucksan, Suwon-si, Gyeonggi-do (KR)
(74) Representative: Greene, Simon Kenneth

(57) **Abstract**

An image forming apparatus is provided. The image forming apparatus includes an electrostatic latent image forming medium forming an electrostatic latent image thereon, a toner supplying unit supplying toner to the electrostatic latent image and developing the electrostatic latent image into a toner image, and a transferring unit transferring the toner image to a printing medium. The electrostatic latent image forming medium includes a frame 110, a thin film transistor (TFT) array 120 stacked on a surface of the frame 110 and including a plurality of TFTs 122a,122b, and a capacitor layer 130 including a common electrode 132 formed on the TFT array 120, a dielectric layer 134 stacked on the common electrode 132, and a plurality of pixel electrodes 136 respectively corresponding to pixels, arranged on a surface of the dielectric layer 134, and connected to drains of the TFTs 122a,122b, respectively. When electric charges are selectively applied to the pixel electrodes 136 via the TFTs 122a,122b, or when electric charges of the pixel electrodes 136 are selectively eliminated via the TFTs 122a,122b, an electrostatic latent image with a potential different from a potential of its surrounding area is formed on the surface of the electrostatic latent image forming medium.

## Description

The present invention relates to an image forming apparatus, and more particularly, to an electrostatic latent image forming medium that forms an electrostatic latent image by using a thin film transistor (TFT) array, and an image forming apparatus printing a desired image by developing the electrostatic latent image.

In general, an electrophotographic image forming apparatus, such as a copier, a laser printer, or a facsimile, prints an image by forming an electrostatic latent image on a photosensitive medium, such as a photosensitive drum or a photosensitive belt, using a laser scanning unit (LSU), developing the electrostatic latent image using a developing agent having a predetermined color, and transferring the developed image onto a sheet of paper.

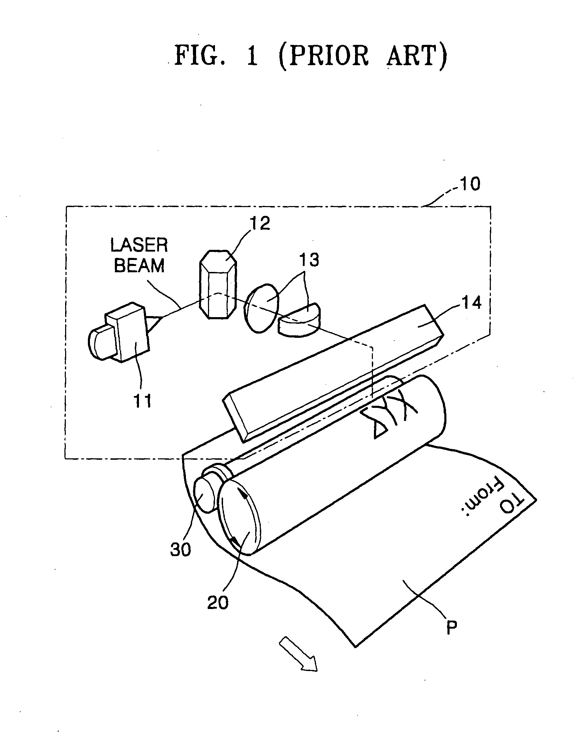

FIG. 1 is a schematic view of a conventional electrophotographic image forming apparatus including an LSU 10, a photosensitive drum 20 on which an electrostatic latent image is formed by the LSU 10 scanning a laser beam onto a surface thereof, and a toner supplying roller 30 supplying toner to the electrostatic latent image formed on the surface of the photosensitive drum 20.

The LSU 10 includes a laser diode (LD) 11 emitting a laser beam, a polygon mirror 12 scanning the laser beam emitted from the LD 11, a focusing lens 13 focusing the laser beam reflected by the polygon mirror 12, and a reflective mirror 14 reflecting the laser beam that passed through the focusing lens 13 to form an electrostatic latent image on the surface of the photosensitive drum 20.

When the LSU 10 scans a laser beam onto the surface of the photosensitive drum 20 charged with a predetermined potential, electric charges in a portion of the surface of the photosensitive drum 20 onto which the laser beam is scanned disappear. Therefore, an electrostatic latent image with a potential different from potentials of other portions of the surface of the photosensitive drum 20 is formed in the portion onto which the laser beam is scanned. Toner supplied by the toner supplying roller 30 is selectively adhered to the electrostatic latent image by an electrostatic force. Thus, the electrostatic latent image develops into a desired image. The developed image on the surface of the photosensitive drum 20 is transferred to a sheet of print paper P and then fixed on the sheet of print paper P by a fixing unit (not shown).

Since the LSU 10 has a complicated structure, the size and manufacturing cost of the conventional image forming apparatus increase. In addition, since the LSU 10 scans a laser beam while the polygon mirror 12 is rotated by a motor (not shown), it is difficult to increase a printing speed by reducing the time spent on scanning the laser beam.

According to an aspect of the present invention, there is provided anelectrostatic latent image forming medium for forming an electrostatic latent image thereon and included in an image forming apparatus. The electrostatic latent image forming medium comprising: a frame; a thin film transistor array stacked on a surface of the frame and comprising a plurality of thin film transistors; and a capacitor layer including a common electrode formed on the thin film transistor array, a dielectric layer stacked on the common electrode, and a plurality of pixel electrodes respectively corresponding to pixels, arranged on a surface of the dielectric layer, and connected to drains of the thin film transistors, respectively.

According to another aspect of the present invention, there is provided an image forming apparatus including: an electrostatic latent image forming medium forming a latent electrostatic image thereon; a toner supplying unit supplying toner to the electrostatic latent image and developing the electrostatic latent image into a toner image; and a transferring unit transferring the toner image to a printing medium, wherein the electrostatic latent image forming medium comprises a frame; a thin film transistor array stacked on a surface of the frame and comprising a plurality of thin film transistors; and a capacitor layer comprising a common electrode formed on the thin film transistor array, a dielectric layer stacked on the common electrode, and a plurality of pixel electrodes respectively corresponding to pixels, arranged on a surface of the dielectric layer, and connected to drains of the thin film transistors, respectively.

A surface of the electrostatic latent image forming medium may be charged with a predetermined potential in advance, sources of the thin film transistors may be grounded, and a voltage may be selectively applied to gates of the thin film transistors. In this case, the electrostatic latent image may be formed by applying a voltage to a gate of a selected thin film transistor to electrically connect a source with a drain of the selected thin film transistor and eliminating electric charges of the pixel electrode connected to the drain.

The thin film transistors may be formed such that a voltage can be selectively applied to their gates and sources. In this case, the electrostatic latent image may be formed by applying a voltage to the gate and the source of the selected thin film transistor and injecting electric charges to the pixel electrode connected to the drain of the selected thin film transistor.
The common electrode may be grounded and formed as a layer covering an entire bottom surface of the dielectric layer. A protective layer may be formed on a surface of the capacitor layer, and the protective layer may be formed of an abrasion resistant material. The electrostatic latent image forming medium may be a rotating drum or a belt circulating along a predetermined path.

The latent electrostatic image forming medium may further include a charging unit charging a surface of the electrostatic latent image forming medium with a predetermined potential in advance. The charging unit may be a charging roller supplying electric charges to the surface of the electrostatic latent image forming medium while rotating in contact with the surface of the electrostatic latent image forming medium.

The toner supplying unit may be a toner supplying roller adhering toner to the electrostatic latent image formed on the surface of the electrostatic latent image forming medium while rotating in contact with the toner stored in a toner container.

The transferring unit may be a transferring roller rotatably disposed parallel to the electrostatic latent image forming medium such that the printing medium can pass between the transferring roller and the electrostatic latent image forming medium.

The image forming apparatus may further include a cleaning unit removing the toner remaining on the surface of the electrostatic latent image forming medium. The cleaning unit may be a cleaning blade contacting the surface of the electrostatic latent image forming medium.

Thus, the present invention provides an electrostatic latent image forming medium forming an electrostatic latent image using a thin film transistor (TFT) array.

The present invention also provides an image forming apparatus including the electrostatic latent image forming medium, which has a fast printing speed, high resolution, and reduced size.

The above and other features and advantages of the present invention will become more apparent by describing in detail exemplary embodiments thereof with reference to the attached drawings in which:
FIG. 1 is a schematic view of a conventional electrophotographic image forming apparatus including a laser scanning unit (LSU);
FIG. 2 is a schematic view of an image forming apparatus according to an embodiment of the present invention;
FIG. 3 is a perspective view of a rotating drum as an electrostatic latent image forming medium illustrated in FIG. 2;
FIGS. 4 and 5 are sectional views of the rotating drum illustrated in FIG. 2 according to an embodiment of the present invention. FIG. 4 is a sectional view of the rotating drum before an electrostatic latent image is formed thereon. FIG. 5 is a sectional view of the rotating drum after an electrostatic latent image is formed thereon; and
FIGS. 6 and 7 are sectional views of the rotating drum illustrated in FIG. 2 according to another embodiment of the present invention. FIG. 6 is a sectional view of the rotating drum before an electrostatic latent image is formed thereon. FIG. 7 is a sectional view of the rotating drum after an electrostatic latent image is formed thereon.

The present invention will now be described more fully with reference to the accompanying drawings, in which exemplary embodiments of the invention are shown. The invention may, however, be embodied in many different forms and should not be construed as being limited to the embodiments set forth therein; rather, these embodiments are provided so that this disclosure will be thorough and complete, and will fully convey the concept of the invention to those skilled in the art. Like reference numerals in the drawings denote like elements, and thus their description will omitted.

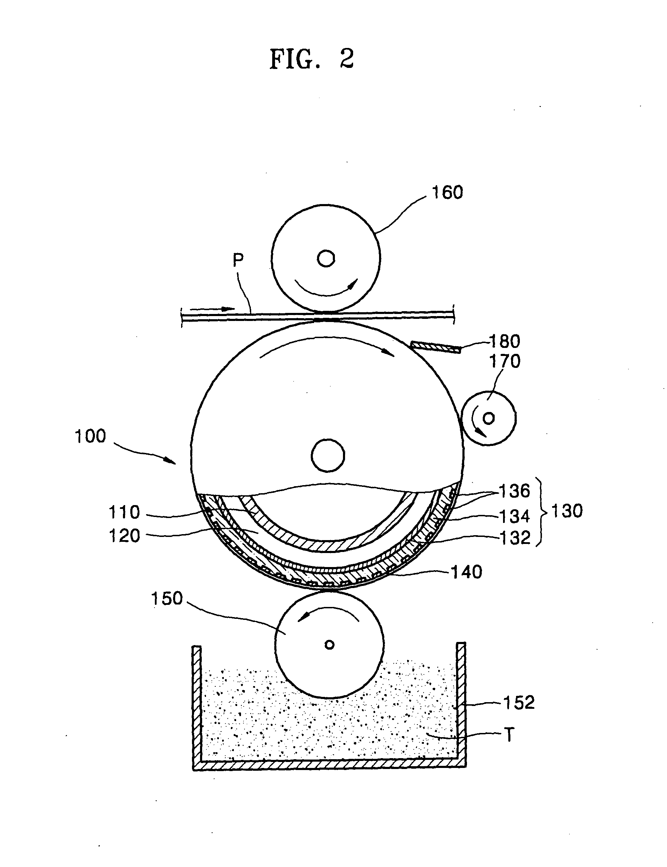

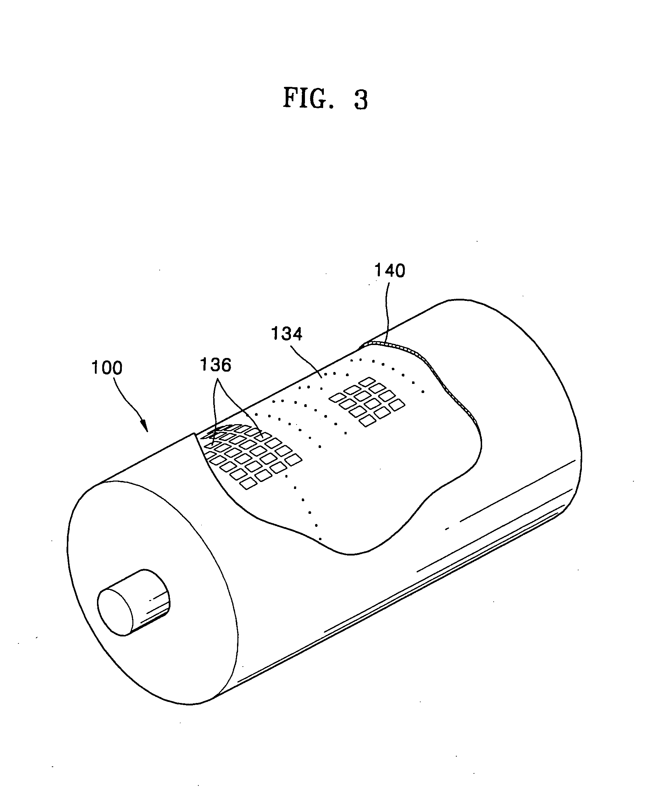

FIG. 2 is a schematic view of an image forming apparatus according to an embodiment of the present invention. FIG. 3 is a perspective view of a rotating drum 100 as an electrostatic latent image forming medium illustrated in FIG. 2.

Referring to FIGS. 2 and 3, the image forming apparatus includes the electrostatic latent image forming medium, a toner supplying unit, and a transferring unit. The image forming apparatus may further include a charging unit depending on how an electrostatic latent image is formed.

The electrostatic latent image forming medium forms an electrostatic latent image that corresponds to an image to be printed thereon. A rotating drum 100 may be used as the electrostatic latent image forming medium.

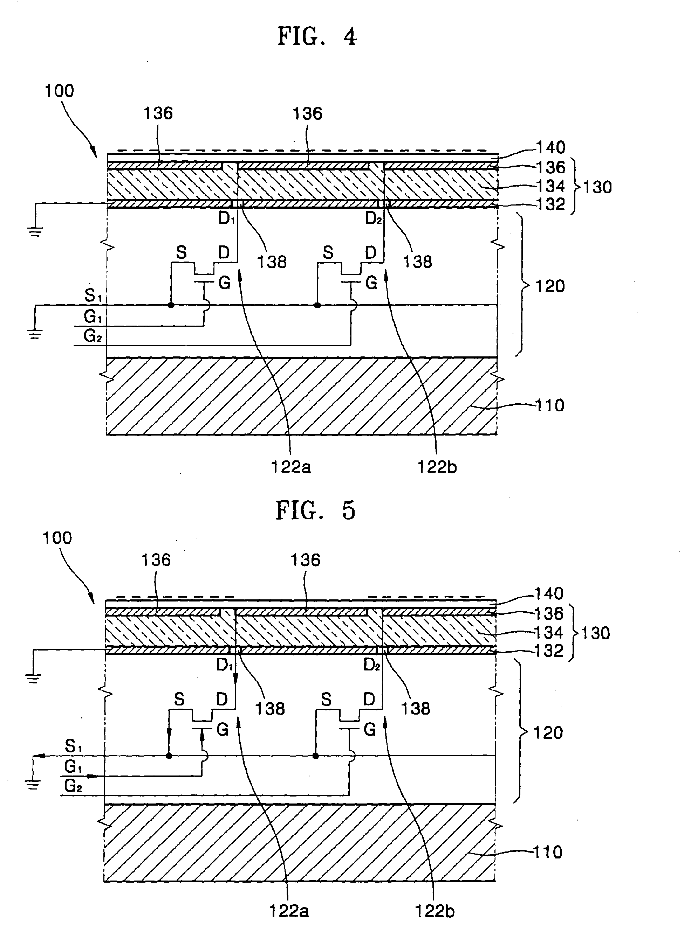

The rotating drum 100 includes a thin film transistor (TFT) array 120 and a capacitor layer 130 stacked sequentially on a surface of a cylindrical frame 110. The TFT array 120 is stacked on the surface of the frame 110 and includes a plurality of TFTs 122a and 122b presented in FIG. 4. The capacitor layer 130 includes a common electrode 132 formed on the TFT array 120, a dielectric layer 134 formed on the common electrode 132, and a plurality of pixel electrodes 136 arranged on a surface of the dielectric layer 134. The number of the pixel electrodes 136 depends on the degree of resolution required by the image forming apparatus. In other words, the number of the pixel electrodes 136 is the same as the number of pixels of an image. An array of the pixel electrodes 136 is formed on the surface of the dielectric layer 134 as illustrated in FIG. 3. A protective layer 140 protecting the dielectric layer 134 and the pixel electrodes 136 may be formed on a surface of the capacitor layer 130, i.e., surfaces of the dielectric layer 134 and the pixel electrodes 136.

The sectional structure of the rotating drum 100 will later be described in detail with reference to FIG. 4. Although in the present embodiment, the rotating drum 100 is used as an electrostatic latent image forming medium, a belt circulating along a predetermined path may also be used as the electrostatic latent image forming medium.

The toner supplying unit supplies toner T to an electrostatic latent image formed on a surface of the rotating drum 100 and develops the electrostatic latent image into a toner image. A variety of devices known to those skilled in the art may be used as the toner supplying unit. For example, a toner supplying roller 150 rotating in contact with the toner T stored in a toner container 152 may be used as illustrated in FIG. 2. The toner supplying roller 150 adheres the toner T stored in the toner container 152 to the surface of the rotating drum 100 while rotating in synchronization with the rotating drum 100.

Generally, a predetermined potential is applied to the toner supplying roller 150. Therefore, the toner T adhered to a surface of the toner supplying roller 150 is moved and adhered to an electrostatic latent image on the rotating drum 100 by a difference between a potential of the surface of the toner supplying roller 150 and a potential of the electrostatic latent image.

The transferring unit transfers the developed toner image on the surface of the rotating drum 100 to a printing medium, for example, a sheet of paper P. A transferring roller 160 may be used as the transferring unit. The transferring roller 160 is disposed parallel to the rotating drum 100 such that it can rotate simultaneously with the rotating drum 100. The sheet of paper P passes between the transferring roller 160 and the rotating drum 100. In this process, the developed toner image on the surface of the rotating drum 100 is transferred to the sheet of paper P.

The transferring unit may include an intermediate transferring belt (not shown). In this case, the developed toner image on the surface of the rotating drum 100 is initially transferred to the intermediate transferring belt. Then, the developed toner image is transferred to the sheet of paper from the intermediate transferring belt by the transferring roller 160. Most of the image forming apparatuses for printing color images include intermediate transferring belts as transferring units. If an intermediate transferring belt is included in an image forming apparatus, a plurality of rotating drums 100 are arranged in series along a path of the intermediate transferring belt.

The charging unit charges the surface of the rotating drum 100 with a predetermined potential. A charging roller 170 supplying electric charges to the surface of the rotating drum 100 while rotating in contact with the surface of the rotating drum 100 may be used as the charging unit. Alternatively, a corona wire may be used as the charging unit.

A cleaning unit removing the toner T remaining on the surface of the rotating drum 100 after the toner image is transferred to the sheet of paper P may be placed near the rotating drum 100. A cleaning blade 180 contacting the surface of the rotating drum 100 may be used as the cleaning unit.

FIGS. 4 and 5 are sectional views of the rotating drum 100 illustrated in FIG. 2 according to an embodiment of the present invention. FIG. 4 is a sectional view of the rotating drum 100 before an electrostatic latent image is formed thereon. FIG. 5 is a sectional view of the rotating drum 100 after an electrostatic latent image is formed thereon. Referring to FIG. 4, the rotating drum 100 includes the frame 110, the TFT array 120, and the capacitor layer 130 stacked sequentially as described above.

The TFT array 120 is stacked on the surface of the frame 110 and includes the TFTs 122a and 122b arranged uniformly. The sources S of the TFTs 122a and 122b are grounded via a source electrode line S₁, and the gates G of the same are connected to gate electrode lines G1 and G2, respectively, such that voltages can be selectively applied to the gates G. The drains D of the TFTs 122a and 122b are connected to the pixel electrodes 136 via drain electrode lines D₁ and D₂, respectively.

As described above, the capacitor layer 130 is formed on the TFT array 120 and includes the common electrode 132, the dielectric layer 134, and the pixel electrodes 136 stacked sequentially. The common electrode 132 is grounded and may be formed as a layer covering the entire bottom surface of the dielectric layer 134. A plurality of holes 138 are formed in the common electrode 132 to insulate the common electrode 132 from the drain electrode lines D₁ and D₂. The pixel electrodes 136 corresponding to pixels, respectively, are formed on the dielectric layer 134 and electrically connected to the drains D of the TFTs 122a and 122b via the drain electrode lines D₁ and D₂, respectively.

The protective layer 140 protecting the dielectric layer 134 and the pixel electrodes 136 may be formed on the capacitor layer 130 and may be formed of an abrasion resistant material.

Even though a circulating belt is used as an electrostatic latent image forming medium, its sectional structure is the same as that of the rotating drum 100 described above.

Hereinafter, a process of forming an electrostatic latent image on the surface of the rotating drum 100 will be described. Referring to FIG. 4, before forming an electrostatic latent image on the surface of the rotating drum 100, the surface of the rotating drum 100 is charged with a predetermined potential using the charging roller 170 of FIG. 2. At this moment, voltages are not applied to the gates G of the TFTs 122a and 122b in the TFT array 120.

FIG. 4 illustrates the rotating drum 100, the surface of which is charged with a negative potential. However, the surface of the rotating drum 100 may be charged with a positive potential. The surface potential of the rotating drum 100 depends on polarity of the potential of the toner T used in an image forming apparatus. In other words, if toner T charged with a negative potential is used, the surface of the rotating drum 100 is charged with negative potential. Conversely, if toner T charged with a positive potential is used, the surface of the rotating drum 100 is charged with the positive potential.

Referring to FIG. 5, voltages are selectively applied to the gates G of the TFTs 122a and 122b. For example, if a voltage is applied to the gate G of the TFT 122a, the source S and the drain D of the TFT 122a are electrically connected. Accordingly, electric charges on the surface of the pixel electrode 136 connected to the drain D of the TFT 122a drain out via the drain electrode line D₁ and the grounded source electrode line S₁.

As described above, by selectively addressing the TFTs 122a and 122b, the surface potential of the rotating drum 100 can be controlled per each pixel electrode 136. Therefore, an electrostatic latent image with a potential different from a potential of its surrounding area is formed on the surface of the rotating drum 100. A potential difference between the electrostatic latent image and the toner supplying roller 150 and a potential difference between the surrounding area of the electrostatic latent image and the toner supplying roller 150 are different. Therefore, the toner T with a predetermined potential is adhered only to the electrostatic latent image, thereby forming a toner image.

As described above, according to the present invention, even without using a conventional LSU, an electrostatic latent image can be formed on the surface of the rotating drum 100 using the TFT array 120. Hence, the configuration of the image forming apparatus is simplified, resulting in a reduction in its size and manufacturing cost. Further, according to the present invention, an entire electrostatic latent image can be formed electrically simultaneously. Therefore, the image forming apparatus according to the present invention can perform printing at a fast speed since the time spent on scanning a laser beam, which was previously performed per line, can be reduced.

Moreover, since the resolution of a conventional image forming apparatus using an LSU depends on the size of an optical spot, it is difficult to improve the resolution. However, the present invention can achieve high resolution since the pixel electrodes 136 can be formed in a very small size using a micro manufacturing process used in a semiconductor fabrication process.

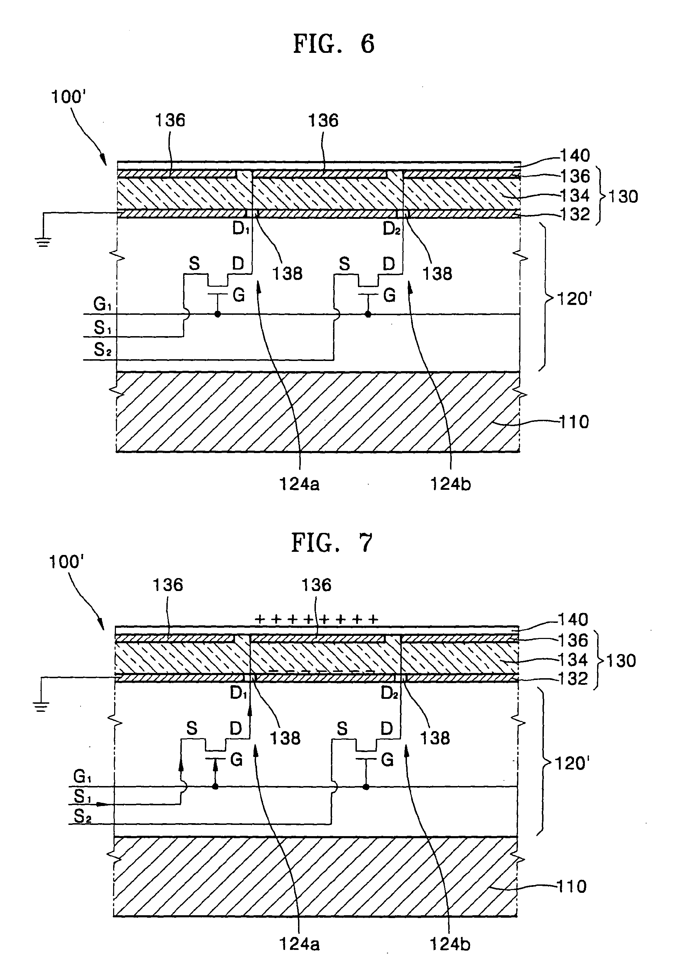

FIGS. 6 and 7 are sectional views of a rotating drum 100' illustrated in FIG. 2 according to another embodiment of the present invention. FIG. 6 is a sectional view of the rotating drum 100' before an electrostatic latent image is formed thereon. FIG. 7 is a sectional view of the rotating drum 100' after an electrostatic latent image is formed thereon. Since the sectional structure of the rotating drum 100' of FIGS. 6 and 7 are identical to that of the rotating drum 100 of FIG. 4 except for wirings of TFTs 124a and 124b, a detailed description will be focused on the difference between them.

Referring to FIG. 6, the rotating drum 100' includes a TFT array 120' stacked on a surface of a frame 110 and the TFTs 124a and 124b arranged uniformly in the TFT array 120'.

Voltages are applied to the gates G of the TFTs 124a and 124b via a common gate electrode line G₁. The sources S of the TFTs 124a and 124b are connected to source electrode lines S₁ and S₂, respectively, such that voltages can be selectively applied to the sources S. The drains D of the TFTs 124a and 124b are connected to a plurality of pixel electrodes 136 via drain electrode lines D₁ and D₂, respectively.

A capacitor layer 130 is stacked on the TFT array 120'. The pixel electrodes 136 formed on the capacitor layer 130 are electrically connected to the drains D of the TFTs 124a and 124b via the drain electrode lines D₁ and D₂, respectively.

A process of forming an electrostatic latent image on the surface of the rotating drum 100' with such a sectional structure will now be described. Referring to FIG. 6, since it is not necessary to charge the surface of the rotating drum 100' in advance with a predetermined potential, an image forming apparatus using the rotating drum 100' does not include the charging roller 170 illustrated in FIG. 2.

Referring to FIG. 7, voltages are selectively applied to the gates G and sources S of the TFTs 124a and 124b. If a voltage is applied to the gate G and the source S of the TFT 124a, electric charges are injected into the pixel electrode 136 connected to the drain D of the TFT 124a via the drain D. The electric charges thus injected are stored in a portion of the capacitor layer 130 corresponding to the pixel electrode 136. Accordingly, the portion storing the electric charges and portions without electric charges coexist on the surface of the rotating drum 100', and these portions have different potentials.

As described above, by injecting electric charges into the pixel electrodes 136 through the selective addressing of the TFTs 124a and 124b, the surface potential of the rotating drum 100' can be controlled per pixel electrode 136. Therefore, an electrostatic latent image corresponding to an image to be printed can be formed. A potential difference between the electrostatic latent image thus formed and the toner supplying roller 150 and a potential difference between the surrounding area of the electrostatic latent image and the toner supplying roller 150 are different. Therefore, the toner T with a predetermined potential is adhered only to the electrostatic latent image, thereby forming a toner image.

The rotating drum 100' in such a structure illustrated in FIGS. 6 and 7 may achieve the effects described above.

As described above, an image forming apparatus according to the present invention forms an electrostatic latent image on a surface of an electrostatic latent image forming medium by using a TFT array included in the electrostatic latent image forming medium. Hence, the image forming apparatus has a simpler configuration than a conventional image forming apparatus including a complicated LSU, resulting in a reduction in size and manufacturing cost.

Further, according to the present invention, an entire electrostatic latent image can be formed electrically. Therefore, a fast printing speed can be achieved since the time spent on scanning a laser beam can be reduced.

Moreover, unlike a conventional image forming apparatus whose resolution depends on the size of an optical spot, the image forming apparatus according to the present invention can achieve high resolution by forming a plurality of pixel electrodes in a very small size using a micro manufacturing process.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. An electrostatic latent image forming medium for forming an electrostatic latent image thereon and included in an image forming apparatus, the electrostatic latent image forming medium comprising:
a frame (110);
a thin film transistor array (120) stacked on a surface of the frame (110) and comprising a plurality of thin film transistors (122a,122b); and
a capacitor layer (130) comprising a common electrode (132) formed on the thin film transistor array (120), a dielectric layer (134) stacked on the common electrode (132), and a plurality of pixel electrodes (136) respectively corresponding to pixels, arranged on a surface of the dielectric layer (134), and connected to drains of the thin film transistors (122a,122b), respectively.

2. The image forming apparatus of claim 1, wherein the latent electrostatic image forming medium further comprises a charging unit arranged to charge a surface of the electrostatic latent image forming medium with a predetermined potential, sources of the thin film transistors (122a,122b) are grounded, and a voltage is selectively applied to gates of the thin film transistors (122a,122b).

3. The image forming apparatus of claim 2, wherein the charging unit is a charging roller (170) arranged to supply electric charges to the surface of the electrostatic latent image forming medium while rotating in contact with the surface of the electrostatic latent image forming medium.

4. The electrostatic latent image forming medium of claim 2, wherein a selected thin film transistor (122a) is arranged to electrically connect a source with a drain of the selected thin film transistor (122a) when a voltage is applied to the gate of the selected thin film transistor (122a) and to eliminate electric charges of the pixel electrode connected to the drain such that an electrostatic latent image is formed.

5. The electrostatic latent image forming medium of any preceding claim, wherein the thin film transistors (122a,122b) are arranged such that a voltage can be selectively applied to their gates and sources.

6. The electrostatic latent image forming medium of claim 4, wherein the gate, the source, and the pixel electrode connected to the drain of the selected thin film transistor (122a) are arranged such that an electrostatic latent image is formed when a voltage is applied to the gate and the source of the selected thin film transistor (122a) and electric charge are injected are injected to the pixel electrode (132).

7. The electrostatic latent image forming medium of any preceding claim, wherein the common electrode is arranged to be electrically grounded and to be a layer covering an entire bottom surface of the dielectric layer (134).

8. The electrostatic latent image forming medium of any preceding claim, further comprising a protective layer (140) on a surface of the capacitor layer (130).

9. The electrostatic latent image forming medium of claim 8, wherein the protective layer (140) is an abrasion resistant material.

10. The electrostatic latent image forming medium of any preceding claim, wherein the electrostatic latent image forming medium is a rotating drum (100).

11. The electrostatic latent image forming medium of any preceding claim, wherein the electrostatic latent image forming medium is a belt circulating along a predetermined path.

12. Use of the electrostatic latent image forming medium of any preceding claim 1, wherein a surface of the electrostatic latent image forming medium is charged with a predetermined potential in advance, sources of the thin film transistors (122a,122b) are grounded, and a voltage is selectively applied to gates of the thin film transistors (122a,122b).

13. Use of the electrostatic latent image forming medium of any preceding claim, wherein the electrostatic latent image is formed by applying a voltage to a gate of a selected thin film transistor (122a) to electrically connect a source with a drain of the selected thin film transistor (122a) and eliminating electric charges of the pixel electrode (136) connected to the drain.

14. Use of the electrostatic latent image forming medium according to any preceding claim, wherein the latent electrostatic image is formed by applying a voltage to the gate and the source of the selected thin film transistor (122a) and injecting electric charges to the pixel electrode (136) connected to the drain of the selected thin film transistor (122a).

15. Use of the electrostatic latent image forming medium according to any preceding claim, wherein the common electrode (132) is grounded and formed as a layer covering an entire bottom surface of the dielectric layer (134).

16. Use of the electrostatic latent image forming medium according to any preceding claim, wherein a protective layer is formed on a surface of the capacitor layer (130).

17. Use of the electrostatic latent image forming medium according to any preceding claim, wherein the protective layer (140) is formed of an abrasion resistant material.

18. An image forming apparatus comprising:
an electrostatic latent image forming medium forming a latent electrostatic image thereon;
a toner supplying unit arranged to supply toner to the electrostatic latent image and to develop the electrostatic latent image into a toner image; and
a transferring unit arranged to transfer the toner image to a printing medium, wherein the electrostatic latent image forming medium comprises an electrostatic latent image forming medium as claimed in any of claims 1 to 11.

19. The image forming apparatus of claim 18, wherein the toner supplying unit is a toner supplying roller (150) arranged to adhere toner to the electrostatic latent image formed on the surface of the electrostatic latent image forming medium while rotating in contact with the toner stored in a toner container (152).

20. The image forming apparatus of any of claims 18 or 19, wherein the transferring unit is a transferring roller (160) rotatably disposed parallel to the electrostatic latent image forming medium such that the printing medium can pass between the transferring roller (160) and the electrostatic latent image forming medium.

21. The image forming apparatus of any of claims 18 to 20, further comprising a cleaning unit arranged to remove the toner remaining on the surface of the electrostatic latent image forming medium.

22. The image forming apparatus of claim 21, wherein the cleaning unit is a cleaning blade (180) arranged to contact the surface of the electrostatic latent image forming medium.
